# EUROPEAN PATENT SPECIFICATION

(11) **EP 3 077 484 B1**
(45) Date of publication and mention of the grant of the patent: **01.04.2020**
(21) Application number: 14816034.4
(22) Date of filing: 02.12.2014
(51) Int. Cl.: C10G 55/06, C10G 11/18, C10G 21/00

(54) **INTEGRATED SOLVENT-DEASPHALTING AND FLUID CATALYTIC CRACKING PROCESS FOR LIGHT OLEFIN PRODUCTION**
INTEGRIERTES VERFAHREN ZUR ENTASPHALTIERUNG UND ZUM KATALYTISCHEN CRACKEN ZUR HERSTELLUNG VON LEICHTEN OLEFINEN
PROCÉDÉ INTÉGRÉ DE DÉSASPHALTAGE ET DE CRAQUAGE CATALYTIQUE FLUIDE DE PRODUCTION D'OLÉFINES LÉGÈRES

(30) Priority: 02.12.2013 US 201361910676 P
(43) Date of publication of application: 12.10.2016
(73) Proprietor: Saudi Arabian Oil Company, Dhahran 31311 (SA)
(72) Inventor: SHAFI, Raheel, Manama (BH); BOURANE, Abdennour, Ras Tanura (SA); AL-GHRAMI, Musaed Salem, Dhahran 31311 (SA); ABUDAWOUD, Raed, Dhahran 31311 (SA)
(74) Representative: Masala, Gian Tomaso
(86) International application number: PCT/US2014/068034
(87) International publication number: WO 2015/084779

(56) References cited:
- NL-C- 79 903
- US-A- 3 628 975
- US-A- 4 686 028
- US-A- 5 000 838
- US-A1- 2003 006 168
- US-B1- 6 190 536

## Description

### RELATED APPLICATIONS

The present application claims the benefit of U.S. Provisional Patent Application No. 61/910,676 filed on 02 December 2013.

### BACKGROUND OF THE INVENTION

### Field of the Invention

The present invention relates to an integrated solvent deasphalting and high-severity catalytic cracking process for the production of light olefins, particularly propylene.

### Description of Related Art

Light olefins are basic intermediates for a large number of processes in the petrochemical industry. They are typically obtained through the thermal cracking (or steam pyrolysis) of petroleum gases and distillates such as naphtha, kerosene or in some instances gas oil. However, thermal cracking typically yields higher rates of ethylene than it does propylene, which has significant market demand.

In particular, there are often shortages of propylene which result in uncertainties in the supply of feedstocks, escalating raw material costs and similar commercial detriments. Additionally, due to uncertainties in the valuations of different hydrocarbon fractions, alternative feedstocks for propylene production are desirable.

Fluidized catalytic cracking is a well-known process that can be tailored to provide relatively higher selectivity of propylene over ethylene, particularly as compared to steam pyrolysis. Common feeds for fluidized catalytic cracking processes include vacuum gas oil, hydrotreated bottoms, hydrocracked bottoms and crude oil residuum. However, these feeds do not typically result in high olefin product yields and require costly pretreatment steps to increase the hydrogen content.

Therefore, a need remains for improved processes and systems for high olefin product yields while minimizing the need to use valuable hydrocarbon fractions from within a refinery.

NL 79903 discloses a method for removing asphalt or other undesirable substances from mineral oil, such as residual oil.

U.S. Patent No. 5,000,838 discloses a fluidized catalyst cracking process which cannot produce light olefins.

U.S. Patent No. 3,628,975 discloses treating heaving oil produced in catalytic cracking processes by reacting these with HF soluble oils to produce polymers.

U.S. Patent No. 4,686,028 discloses deasphalting hydrocarbon feed to form components which include resin, and upgrading the resin fraction.

U.S. Patent No. 6,190,536 discloses catalytic cracking processes in which a wet gas stream is produced, which is then processed to separate light olefin gases.

U.S. 2003/0006168 discloses increasing production of light olefins in catalytic cracking by using a particular catalyst.

### SUMMARY OF THE INVENTION

It is an objective of the present invention to provide a process as claimed in claim 1 and in the other appended claims in which alternative feedstocks are cracked using fluidized catalytic cracking to increase the proportion of lighter olefins, in particular propylene.

The process described herein broadly comprehends an integrated process that permits the use of straight run residual fractions as a feedstock to produce lighter olefins including propylene.

For the process described herein a new type of feedstream is provided to the integrated system and process in order to maximize light olefins production. A blend of natural gas condensate, and heavy oil residue(s), is catalytically cracked to produce a light olefin-rich product stream. In particular, the light natural gas condensate is used as both solvent in a solvent-deasphalting unit and a portion for the feedstock to a fluidized catalytic cracking process. In addition, blending a heavy boiling hydrocarbon stream with a light natural gas condensate stream enables efficient cracking in fluidized catalytic cracking process processes while sustaining the heat balance within the cracking unit.

Before being catalytically cracked to a stream rich in light olefins, the light crude oil fractions, such as natural gas condensates, are used as the solvent in a solvent deasphalting unit, where the metallic and asphaltenic compounds, that could poison the fluidized catalytic cracking catalysts, are removed from the heavy residual oil fraction (such as atmospheric or vacuum distillation residue). This allows obtaining a mixture of the light hydrocarbon fraction and deasphalted heavy feed that has reduced level amounts of metals, sulfur, nitrogen, ash, and Conradson carbon.

The light streams that are blended in the integrated generally comprise hydrocarbons with carbon numbers in the range of 5-7, where the deasphalted oil is solubilized in the solvent. The insoluble pitch will precipitate out of the mixed solution and is separated from the mixture of solvent and deasphalted/demetalized oil in one or more extractors.

Still other aspects, embodiments, and advantages of these exemplary aspects and embodiments, are discussed in detail below. Moreover, it is to be understood that both the foregoing information and the following detailed description are merely illustrative examples of various aspects and embodiments, and are intended to provide an overview or framework for understanding the nature and character of the claimed aspects and embodiments. The accompanying drawings are included to provide illustration and a further understanding of the various aspects and embodiments, and are incorporated in and constitute a part of this specification. The drawings, together with the remainder of the specification, serve to explain principles and operations of the described and claimed aspects and embodiments.

### BRIEF DESCRIPTION OF THE DRAWINGS

The present process will be described in further detail below and with reference to the attached drawings in which the same or similar reference number is used to refer to the same and similar elements, and in which:
FIG. 1 is a schematic flow diagram of an embodiment of apparatus for fluidized catalytic cracking of a natural gas condensate and/or naptha blended with a heavy oil residue(s) feedstream suitable for production of light olefins including propylene;
FIG. 2 is a simplified schematic illustration of a solvent deasphalting/demetalizing system;
FIG. 3 is a simplified schematic illustration of a downflow fluidized catalytic cracking reactor system; and
FIG. 4 is a simplified schematic illustration of a riser fluidized catalytic cracking reactor system.

### DETAILED DESCRIPTION OF THE INVENTION

Due to increasing demand for propylene, new feedstock sources are desired for its production. The processes and systems described herein utilize feedstock sources for fluidized catalytic cracking including natural gas condensates and petroleum naphthas, along with residual fractions, directly from atmospheric or vacuum distillation bottoms, in an integrated process including solvent deasphalting and fluidized catalytic cracking. The integrated process maximizes the yield of light olefins, including propylene.

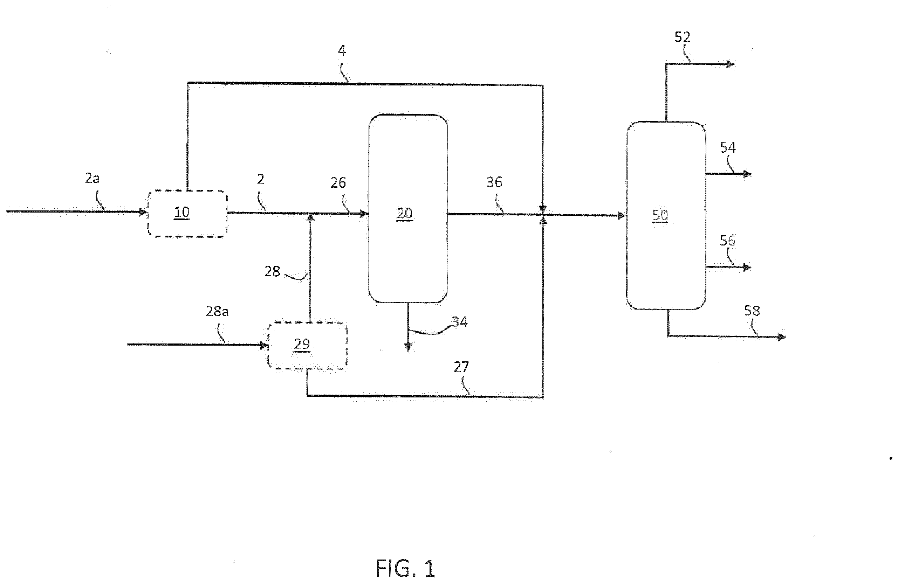

FIG. 1 is a schematic flow diagram including integrated solvent deasphalting and fluidized catalytic cracking processes. The system generally includes a solvent deasphalting/demetalizing zone **20** and a fluidized catalytic cracking reaction and separation zone **50.**

In certain embodiments, an initial feedstock **2** is charged to the solvent deasphalting zone **20** along with a solvent feedstock **28** in which it is subjected to conventional solvent deasphalting processes, and an asphalt phase is discharged via outlet **34.** The deasphalted/demetalized oil, in combination with the solvent feedstock that is used for deasphalting operations in place of conventional solvent that is typically recycled from the system, is passed via a stream **36** to the fluidized catalytic cracking reaction and separation zone **50.** Fluidized catalytic cracking reactions occur under conditions that maximize formation of light olefins, including propylene, and products including light gases **52,** olefins **54,** gasoline **56** and cycle oil **58** are recovered from a separation zone.

In certain embodiments, an optional splitter **10** is provided which receives all or a portion of an initial feedstock **2a** and is in fluid communication with the fluidized catalytic cracking reaction and separation zone **50** to pass a light fraction **4** thereto and the solvent deasphalting zone **20** to pass a heavy fraction **2** thereto. In these embodiments the light fraction **4** is recombined with stream **36** containing deasphalted/demetalized oil and solvent feedstock, and is charged to the fluidized catalytic cracking reaction and separation zone **50.**

In certain embodiments, an optional splitter **29** is provided which receives all or a portion of the initial solvent feedstock **28a** and is in fluid communication with the fluidized catalytic cracking reaction and separation zone **50** to pass a heavy fraction **27** of the solvent feedstock thereto and the solvent deasphalting zone **20** to pass a light fraction **28** thereto. In these embodiments the light fraction **28** serves as the solvent in the solvent deasphalting/demetalizing zone **20** and the heavy fraction **27** is combined with stream **36** containing deasphalted/demetalized oil and solvent feedstock, and is charged to the fluidized catalytic cracking reaction and separation zone **50.**

In additional embodiments (not shown), a relatively small portion of stream **36,** particularly all or a portion of C5 and lower components, can be recycled to solvent deasphalting/demetalizing zone **20** or flashed off, since these compounds will not be completely cracked under the herein FCC conditions. For instance, these C5 and lower components derived from Khuff condensate can be recycled or flashed.

Unit operations associated with the fluidized catalytic cracking reaction and separation zone **50** and solvent deasphalting zone **20** are described below. In the interest of clarity, the numerous valves, temperature sensors, electronic process controllers and the like that are customarily employed, and that are well known to those of ordinary skill in the art of solvent deasphalting/demetalizing and fluidized catalyst cracking, are not included in the attached schematic illustration. Accessory systems that are utilized in conventional fluidized catalyst cracking systems such as, for example, air supply, catalyst hoppers, torch oil supply, flue gas handling and heat recovery, fresh and spent catalyst hoppers for storage of make-up and used/equilibrium catalyst that can be added to, or removed from the regenerator, are not shown.

According to the present process and system, the solvent in the solvent deasphalting/demetalizing operation is not separated from the deasphalted/demetalized oil, contrary to conventional solvent deasphalting/demetalizing operations.

Solvent deasphalting/demetalizing is generally carried-out in liquid phase and therefore the temperature and pressure are set accordingly at or below the critical temperature and pressure of the solvent. In a typical two-stage solvent deasphalting/demetalizing process, the temperature is lower in the first stage than that of the second stage to separate the bulk of the asphaltenes. The second stage temperature is set to control the deasphalted/demetalized oil quality and quantity. The temperature has a large impact on the quality and quantity of deasphalted/demetalized oil. An extraction temperature increase will result in a decrease in deasphalted/demetalized oil yield, in turn the deasphalted/demetalized oil will be lighter, less viscous, and contain less metals, asphaltenes, sulfur, and nitrogen. A temperature decrease will have the opposite impact. In general, the deasphalted/demetalized yield decreases with higher quality product by raising the extraction system temperature, and the deasphalted/demetalized yield increases with lower quality product by lowering extraction system temperature.

In conventional solvent deasphalting/demetalizing operations, composition of the solvent is an important process variable. The solubility properties of the solvent increases with increasing critical temperature, C3<iC4<nC4<iC5. An increase in critical temperature of the solvent increases the deasphalted/demetalized oil yield. However, it should be noted that solvents possessing higher critical temperatures have lower selectivity resulting in lower deasphalted/demetalized oil quality. According to certain embodiments of the process herein, in which natural gas condensates are used as a solvent in the solvent deasphalting/demetalizing operation, all or a portion of the heavy-end components of the natural gas condensates are removed by fractionation and the lighter portion of the stream is used in the solvent deasphalting/demetalizing process.

The ratio of the light fraction (solvent feedstock) to the heavy fraction (oil feedstock) entering the deasphalting/demetailzing unit impacts selectivity and to a lesser degree yield of deasphalted/demetalized oil. The major effect of light to heavy stream ratio is that a higher ratio results in a higher quality of the deasphalted/demetalized oil for a given fixed deasphalted/demetalized yield. A high solvent (light fraction) to heavy fraction ratio is preferred in certain embodiments due to of enhanced selectivity. In the process and system herein, ratio of the light to the heavy fraction entering the deasphalting/demetailzing unit is determined as a function of the coke that will be produced during the cracking reaction within the fluidized catalytic cracking reaction, as described further herein. The solvent-to-oil ratio is in the range of 5:1 to 50:1, in certain embodiments in the range of 5:1 to 25:1, and in further embodiments in the range of 5:1 to 15:1. The ratio of the light to heavy fraction of the hydrocarbon mixture fed to the fluidized catalytic cracking process is manipulated not only to ensure generation of sufficient coke in the cracking reactions so as to maintain a heat balance in the fluidized catalytic cracking unit, but also to facilitate optimal conversion to obtain high light olefins yield.

The solvent deasphalting unit is operated at a pressure that is sufficiently high to maintain the solvent in the liquid phase within the extraction system. The pressure is typically considered as a variable and is typically not altered unless the solvent composition varies.

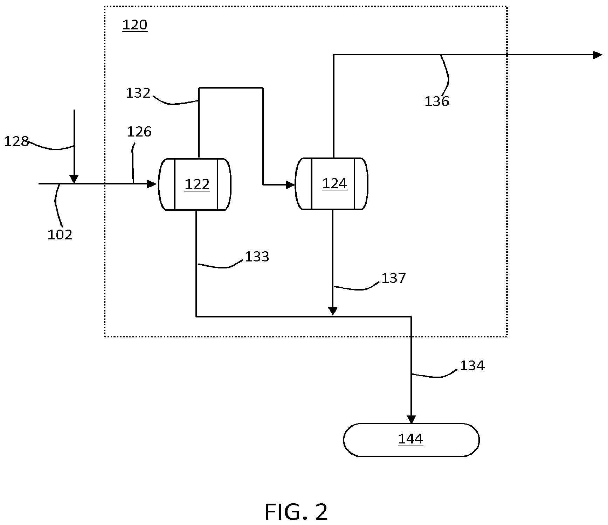

FIG. 2 is a simplified schematic illustration of a solvent deasphalting/demetalizing system. Exemplary unit operations within solvent deasphalting zone **20** of the integrated system described with respect to FIG. 1 are shown. While the system and process is shown with primary and secondary extraction zones, it should be appreciated that one extraction zone can be used, or alternatively more than two extraction zones can be used.

A solvent deasphalting zone **120** typically includes a primary extraction zone **122** and a secondary extraction zone **124.** Primary extraction zone **122** includes an inlet for receiving a combined stream **126** including the hydrocarbon feedstock **102** (or a heavy portion thereof in embodiments in which the optional splitter **10** is used) and a solvent feedstock **128.** Primary extraction zone **122** also includes an outlet for discharging a primary deasphalted/demetalized oil phase **132** and one or more outlets for discharging a primary asphalt phase **133.** Secondary extraction zone **124** typically includes an inlet for receiving the primary deasphalted/demetalized oil phase **132,** an outlet for discharging a secondary deasphalted/demetalized oil phase **136** and an outlet for discharging a secondary asphalt phase **137.** The combined primary asphalt phase **133** and secondary asphalt phase **137,** stream **134,** is passed to an asphalt pool **144.**

According to the process herein, the solvent feedstock **128** is selected so as to be suitable not only as a solvent for facilitating precipitation of the asphalt phase **134** of the feedstock **102,** but also to serve as a reactant in the downstream fluidized catalytic cracking process to yield fluidized catalytic cracking products olefins, gasoline and cycle oil, with a maximized propylene yield. Therefore the solvent deasphalting zone **120** does not require separate solvent recovery/recycle steps or unit operations since the solvent component is part of the combined stream **136** that is the feed to the fluidized catalytic cracking reaction.

In general, during fluidized catalytic cracking processes, petroleum derived hydrocarbons are catalytically cracked with an acidic catalyst maintained in a fluidized state, which is regenerated on a continuous basis. The main product from such processes has conventionally been gasoline. Other products are also produced in smaller quantities via fluidized catalytic cracking such as liquid petroleum gas and cycle oil. Coke deposited on the catalyst is burned off at high temperatures and in the presence of air prior to recycling regenerated catalyst back to the reaction zone. As discussed above, a shift in market demands has led to use of fluidized catalytic cracking for production of light olefins, including propylene, as a primary product. These operations have significant economic advantages, particularly with respect to oil refineries that are highly integrated with petrochemical production facilities.

According to the processes and systems herein, a high-severity fluid catalytic cracking process is used to facilitate conversion of the feed into a product rich in light olefins. For instance, a PetroFCC process (disclosed by UOP, a Honeywell International Inc. company) can be suitable for the present processes and systems. In general, suitable high-severity fluid catalytic cracking processes result in minimization of olefin consuming reactions thereby maximizing the yield of light olefin products such as propylene.

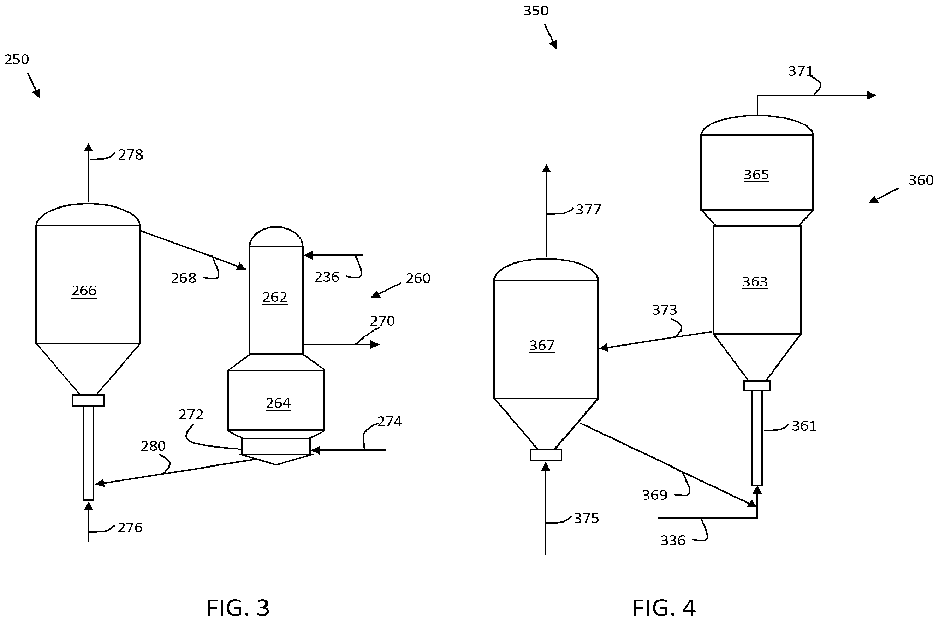

In certain embodiments, a fluidized catalytic cracking unit configured with a downflow reactor is provided that operates under conditions that promote formation of olefins, particularly propylene, and that minimize olefin-consuming reactions including hydrogen-transfer reactions. FIG. 3 is a simplified schematic illustration of a downflow fluidized catalytic cracking unit. Exemplary unit operations within fluidized catalytic cracking zone **50** of the integrated system described with respect to FIG. 1 are shown. A fluidized catalytic cracking unit **250** includes a reactor/separator **260** having a reaction zone **262** and a separation zone **264.** Fluidized catalytic cracking unit **250** also includes a regeneration zone **266** for regenerating spent catalyst. In particular, a charge **236** (which is the combined stream of deasphalted/demetalized and solvent feed, and in certain embodiments recombined with light ends of the feed and/or heavy ends of the solvent as described above) is introduced to the reaction zone, in certain embodiments accompanied by steam or other suitable gas for atomization of the feed (not shown). An effective quantity of heated fresh or hot regenerated solid cracking catalyst particles from regeneration zone 266 is conveyed to the top of reaction zone **262** also transferred, e.g., through a downwardly directed conduit or pipe **268,** commonly referred to as a transfer line or standpipe, to a withdrawal well or hopper (not shown) at the top of reaction zone **262.** Hot catalyst flow is typically allowed to stabilize in order to be uniformly directed into the mix zone or feed injection portion of reaction zone **262.** The charge **236** is injected into a mixing zone through feed injection nozzles typically situated proximate to the point of introduction of the regenerated catalyst into reaction zone **262.** These multiple injection nozzles result in the catalyst and oil mixing thoroughly and uniformly. Once the charge contacts the hot catalyst, cracking reactions occur.

The reaction vapor of hydrocarbon cracked products, unreacted feed and catalyst mixture quickly flows through the remainder of reaction zone **262** and into the rapid separation zone **264** at the bottom portion of reactor/separator **260.** Cracked and uncracked hydrocarbons are directed through a conduit or pipe **270** to a conventional product recovery section known in the art to yield fluidized catalytic cracking products olefins, gasoline and cycle oil, with a maximized propylene yield. If necessary for temperature control, a quench injection can be provided near the bottom of reaction zone **262** immediately before the separation zone **264.** This quench injection quickly reduces or stops the cracking reactions and can be utilized for controlling cracking severity.

The reaction temperature, i.e., the outlet temperature of the downflow reactor, can be controlled by opening and closing a catalyst slide valve (not shown) that controls the flow of regenerated catalyst from regeneration zone **266** into the top of reaction zone **262.** The heat required for the endothermic cracking reaction is supplied by the regenerated catalyst. By changing the flow rate of the hot regenerated catalyst, the operating severity or cracking conditions can be controlled to produce the desired yields of light olefinic hydrocarbons. A stripper **272** is also provided for separating oil from the catalyst, which is transferred to regeneration zone **266.** The catalyst from separation zone **264** flows to the lower section of the stripper **272** that includes a catalyst stripping section into which a suitable stripping gas, such as steam, is introduced through streamline **274.** The stripping section is typically provided with several baffles or structured packing (not shown) over which the downwardly flowing catalyst **280** passes counter- currently to the flowing stripping gas. The upwardly flowing stripping gas, which is typically steam, is used to "strip" or remove any additional hydrocarbons that remain in the catalyst pores or between catalyst particles. The stripped or spent catalyst is transported by lift forces from the combustion air stream **276** through a lift riser of the regeneration zone **264.** This spent catalyst, which can also be contacted with additional combustion air, undergoes controlled combustion of any accumulated coke. Flue gases are removed from the regenerator via conduit **278.** In the regenerator, the heat produced from the combustion of the by-product coke is transferred to the catalyst raising the temperature required to provide heat for the endothermic cracking reaction in the reaction zone **262.** According to the process herein, since the light solvent feedstock is combined with the heavy feedstock as the feed **236,** the solvent to oil ratio in the initial solvent deasphalting/demetalizing process is selected so as to provide sufficient coking of the catalyst to provide the heat balance during regeneration.

In one embodiment, a suitable fluidized catalytic cracking unit **250** that can be employed in the integrated solvent deasphalting and high-severity catalytic cracking system and process described herein can be similar to those described in US Patent Number 6,656,346, and US Patent Publication Number 2002/0195373. Important properties of downflow reactors include introduction of feed at the top of the reactor with downward flow, shorter residence time as compared to riser reactors, and high catalyst to oil ratio, e.g., in the range of 20:1 to 30:1.

In general, the operating conditions for the reactor of a suitable downflow FCC unit include:
reaction temperature of 500°C to 650°C, in certain embodiments 550°C to 630°C, and in further embodiments 570°C to 610°C;
reaction pressure of 1 Kg/cm² to 5 Kg/cm², in certain embodiments of 1 Kg/cm² to 3 Kg/cm², in further embodiments of 1 Kg/cm² to 2 Kg/cm²;
contact time (in the reactor) of 0.1 seconds to 10 seconds, in certain embodiments of 0.1 seconds to 5 seconds, in further embodiments of 0.2 seconds to 0.8 seconds; and
a catalyst to feed ratio of 2:1 to 40:1, in certain embodiments of 2:1 to 30:1, in further embodiments of 2:1 to 20:1.

In certain embodiments, a fluidized catalytic cracking unit configured with a riser reactor is provided that operates under conditions that promote formation of olefins, particularly propylene, and that minimize olefin-consuming reactions including hydrogen-transfer reactions. FIG. 4 is a simplified schematic illustration of a riser fluidized catalytic cracking unit. Exemplary unit operations within fluidized catalytic cracking zone **50** of the integrated system described with respect to FIG. 1 are shown. A fluidized catalytic cracking unit **350** which includes a riser reactor and can be used in the herein integrated solvent deasphalting and high-severity catalytic cracking system and process. Fluidized catalytic cracking unit **350** includes a reactor/separator **360** having a riser portion **361,** a reaction zone **363** and a separation zone **365.** Fluidized catalytic cracking unit **350** also includes a regeneration vessel **367** for regenerating spent catalyst. A charge **336** (which is the combined stream of deasphalted/demetalized and solvent feed, and in certain embodiments recombined with light ends of the feed and/or heavy ends of the solvent as described above) is introduced to the reaction zone, in certain embodiments accompanied by steam or other suitable gas for atomization of the feed (not shown). The charge **336** is admixed and intimately contacted with an effective quantity of heated fresh or regenerated solid cracking catalyst particles which are conveyed via a conduit **369** from regeneration vessel **367.** The feed mixture and the cracking catalyst are contacted under conditions to form a suspension that is introduced into the riser **361.** In a continuous process, the mixture of cracking catalyst and hydrocarbon feedstock proceed upward through the riser **361** into reaction zone **363.** In riser **361** and reaction zone **363,** the hot cracking catalyst particles catalytically crack relatively large hydrocarbon molecules by carbon-carbon bond cleavage.

During the reaction, as is conventional in fluidized catalytic cracking operations, the cracking catalysts become coked and hence access to the active catalytic sites is limited or nonexistent. Reaction products are separated from the coked catalyst using any suitable configuration known in a fluidized catalytic cracking units, generally referred to as the separation zone **365** in a fluidized catalytic cracking unit **350,** for instance, located at the top of the reactor **360** above the reaction zone **363.** The separation zone can include any suitable apparatus known to those of ordinary skill in the art such as, for example, cyclones. The reaction product is withdrawn through conduit **371.** Catalyst particles containing coke deposits from fluid cracking of the hydrocarbon feedstock pass through a conduit **373** to regeneration zone **367.** According to the process herein, since the light solvent feedstock is combined with the heavy feedstock as the feed **336,** the solvent to oil ratio in the initial solvent deasphalting/demetalizing process is selected so as to provide sufficient coking of the catalyst to provide the heat balance during regeneration.

In regeneration zone **367,** the coked catalyst comes into contact with a stream of oxygen-containing gas, e.g., pure oxygen or air, which enters regeneration zone **367** via a conduit **375.** The regeneration zone **367** is operated in a configuration and under conditions that are known in typical a fluidized catalytic cracking operations. For instance, regeneration zone **367** can operate as a fluidized bed to produce regeneration off-gas comprising combustion products which is discharged through a conduit **377.** The hot regenerated catalyst is transferred from regeneration zone **367** through conduit **369** to the bottom portion of the riser **361** for admixture with the hydrocarbon feedstock and noted above.

In one embodiment, a suitable a fluidized catalytic cracking unit **350** that can be employed in the integrated solvent deasphalting and high-severity catalytic cracking system and process described herein can be similar to that described in US Patent Numbers 7,312,370, 6,538,169, and 5,326,465.

In general, the operating conditions for the reactor of a suitable riser fluidized catalytic cracking unit include:
reaction temperature of 500°C to 650°C, in certain embodiments 550°C to 630°C, and in further embodiments 570°C to 610°C;
reaction pressure of 1 Kg/cm² to 5 Kg/cm², in certain embodiments of 1 Kg/cm² to 3 Kg/cm², in further embodiments of 1 Kg/cm² to 2 Kg/cm²;
contact time (in the reactor) of 0.7 seconds to 10 seconds, in certain embodiments of 1 second to 5 seconds, in further embodiments of 1 second to 2 seconds ; and
a catalyst to feed ratio of 2:1 to 40:1, in certain embodiments of 2:1 to 25:1, in further embodiments of 2:1 to 15:1.

The catalyst or catalyst systems that can be used in the process described herein are not limited. A catalyst that is suitable for the particular charge and the desired product is conveyed to the FCC reactor within the FCC reaction and separation zone. In certain embodiments, to promote formation of olefins and minimize olefin-consuming reactions, such as hydrogen-transfer reactions, an FCC catalyst mixture is used in the FCC reaction and separation zone, including an FCC base catalyst and an FCC catalyst additive.

In particular, a matrix of a base cracking catalyst can include one or more clays such as kaolin, montmorilonite, halloysite and bentonite, and/or one or more inorganic porous oxides such as alumina, silica, boria, chromia, magnesia, zirconia, titania and silica-alumina. The base cracking catalyst preferably has a bulk density of 0.5 g/ml to 1.0 g/ml, an average particle diameter of 50 microns to 90 microns, a surface area of 50 m²/g to 350 m²/g and a pore volume of 0.05 ml/g to 0.5 ml/g.

A suitable catalyst mixture contains, in addition to a base cracking catalyst, an additive containing a shape-selective zeolite. The shape selective zeolite referred to herein means a zeolite whose pore diameter is smaller than that of Y-type zeolite, so that hydrocarbons with only limited shape can enter the zeolite through its pores. Suitable shape-selective zeolite components include ZSM-5 zeolite, zeolite omega, SAPO-5 zeolite, SAPO-11 zeolite, SAPO34 zeolite, and pentasil-type aluminosilicates. The content of the shape-selective zeolite in the additive is generally in the range of 20 to 70 wt%, and preferably in the range of 30 to 60 wt%.

The additive preferably has a bulk density of 0.5 g/ml to 1.0 g/ml, an average particle diameter of 50 microns to 90 microns, a surface area of 10 m²/g to 200 m²/g and a pore volume of 0.01 ml/g to 0.3 ml/g.

A percentage of the base cracking catalyst in the catalyst mixture can be in the range of 60 to 95 wt% and a percentage of the additive in the catalyst mixture is in a range of 5 to 40 wt%. If the percentage of the base cracking catalyst is lower than 60 wt% or the percentage of additive is higher than 40 wt%, high light-fraction olefin yield cannot be obtained, because of low conversions of the feed oil. If the percentage of the base cracking catalyst is higher than 95 wt%, or the percentage of the additive is lower than 5 wt%, high light-fraction olefin yield cannot be obtained, while high conversion of the feed oil can be achieved.

The initial heavy feedstock for use in above-described systems and processes can be a straight run residue obtained from atmospheric distillation and/or vacuum distillation. In general, the feedstock contains hydrocarbons having boiling point greater than 380°C in the case of atmospheric residue, and greater than 540°C in the case of vacuum residue. The source of the residues can be crude oil, synthetic crude oil, bitumen, oil sand, shell oil, coal liquids, or a combination including one of the foregoing sources.

In a process using the system of FIG. 1, all or a portion of the light stream 28a (naphtha or natural gas condensate of carbon range starting at C5 or above) is first fed to an optional splitter 29 to remove a heavy portion 27 thereof, if required to optimize the solvent deasphalting/demetalizing operation. The light portion 28 is admixed with a heavy oil fraction 2, such as atmospheric or vacuum residue. The resulting mixture 26 is then transferred to the solvent deasphalting/demetalizing zone 20, for instance, which can be operated in a manner similar to what is reported in B.M. Yezhov, A.S. Aigenson, G.A. Berg, F.Kh. Malikov, Yu.S. Sabadash and G.A. Vorms, Hydrocarbon Separation, including Engineering Developments, 1971, 8th World Petroleum Congress, 221-227, in which a gasoline-type stream is used as the solvent to extract asphaltenes from a heavy hydrocarbon fraction. The asphaltenes 34 are rejected from the hydrocarbon mixture and the resultant mixture 36 is directly sent to the fluidized catalytic cracking zone 50. In embodiments in which the optional splitter is used to remove a heavy portion of the solvent feedstream prior to mixing with the heavy fraction 2, the heavy portion 27 is recombined with the feed passed to the fluidized catalytic cracking zone 50.

Advantageously, the processes and systems described herein accommodate the high demand for light olefins with the use of alternative feedstocks including hydrocarbons in lighter boiling-ranges than those which are usually used as fluidized catalytic cracking feeds. These lighter feeds include natural gas condensates and petroleum naphthas.

The problems that arise when lighter feedstocks are used in the fluidized catalytic cracking feeds process is the low coking on the catalyst. This lower amount of coke is not sufficient to provide the heat for catalytic regeneration in the fluidized catalytic cracking process. Therefore, an additional heat source would typically be required for the fluidized catalytic cracking process to operate properly. However, according to the process herein, blending an effective quantity of a very heavy fraction, such as atmospheric or vacuum residue, with the light fraction(s) can result in sufficient coke for catalyst regeneration.

Using the lighter feeds as blends, however, does not counter the negative impacts that result from use of straight run residues (i.e., those directly from atmospheric an./or vacuum distillation) such as catalyst poisoning due to metal and asphaltenic content of the heavy oil fractions. In order to avoid poisoning the fluidized catalytic cracking catalyst these compounds must be removed from the heavy fraction, which leads to a better overall process performance and longer catalyst life. According to the process herein, these metals and asphaltenes are removed by a tightly integrated solvent deasphalting/demetalizing process, in which the solvent also serves as the feedstock blend to increase propylene yield.

It is to be understood that the present processes contemplate use of the natural gas condensate as a solvent and reactant. This is in contrast to known processes such as that disclosed in Bartilucci et al. US Patent 5,000,838 in which naphtha that may remain in a deasphalted oil does not serving as a reactant to produce light olefins. In that process, the boiling range of the cracking product is 100 - 400 °F (37.8 - 204.4 °C), whereas light olefins (ethylene and propylene) boil in the range of -155 to -54 °F (-103.9 to -47.8 °C) indicating that the fluidized catalytic cracking process described therein is operated at conditions through which light olefins cannot be produced and that any naphtha in the cracking feed does not react at all, since one would expect the boiling range of the product to be less than the feed if cracking reactions were to occur.

### Example

An atmospheric residue derived from atmospheric distillation of a crude oil was mixed with gas condensate at a volumetric condensate:residue ratio of 10:1 for partial deasphalting. The mixture was stirred at room temperature, them maintained for a few hours to settle, and filtered to separate the precipitated material. About 5wt% of the atmospheric residue was precipitated. The properties of the atmospheric residue, condensate and obtained deasphalted mixture are given in Table 1 below.

| Table 1 | | | |
|---|---|---|---|
| | Atmospheric Residue | Gas Condensate | Deasphalted Mixture |
| **Gravity, Specific 60/60 °F** | 0.9651 | 0.7721 | 0.7796 |
| **Sulfur, Total Wt %** | 3.2 | 0.0521 | 0.0560 |
| **Vanadium, ppm** | 29 | - | 1.4 |
| **Microcarbon Residue, wt%** | 6.2 | 0.01 | 0.31 |
| **Nickel, ppm** | 9 | - | <2 |

Catalytic cracking of the deasphalted mixture was conducted using a micro activity testing (MAT) unit well known to a person of ordinary skill in the art at 600°C and a C/O ratio of ∼4. The catalyst contains a mixture of Y zeolite and ZSM-5. The cracking of the gas condensate alone was also similarly performed and the products yields difference (relative) are shown in Table 2. As seen, more coke can be produced from the mixture feed while maintaining propylene yield.

| Table 2 | |
|---|---|
| Product Yields | Deasphalted Mixture Vs Gas condensate relative difference (%) |
| Dry gas | +3 |
| LPG | -6 |
| propylene | -6 |
| gasoline | -1 |
| LCO | +20 |
| HCO | +17 |
| Coke | +28 |

The process and system of the present invention have been described above and in the attached drawings; however, modifications will be apparent to those of ordinary skill in the art and the scope of protection for the invention is to be defined by the claims that follow.

## Claims

1. An integrated process for production of propylene containing light olefins, comprising:
(a) mixing (i) a residual oil feedstream, and (ii) a natural gas condensate solvent, wherein (ii) and (i) are mixed at a ratio of from 5:1 to 50:1, in quantities and under conditions sufficient to precipitate at least a fraction of asphaltenes to produce (iii) a mixture of solvent and deasphalted residual oil, and (iv) precipitated asphaltenes;
(b) subjecting (iii) to fluidized catalytic cracking reactions, wherein both the solvent and the deasphalted residual oil from (iii) serve as reactants in the fluidized catalytic cracking reaction, wherein said fluidized catalytic cracking reactions are carried out:
- in a riser FCC unit, with a catalyst, at a catalyst to (iii) ratio of from 2:1 to 40:1, a reaction temperature of from 500°C to 650°C, a reaction pressure of from 1 kg/cm² to 5 kg/cm², and a contact time of from 0.7 seconds to 10 seconds, or
- in a downflow FCC unit with a catalyst, at a catalyst to (iii) ratio of from 2:1 to 40:1, a reaction temperature of from 500°C to 650°C, a reaction pressure of from 1 kg/cm² to 5 kg/cm², and a contact time from 0.1 second to 10 seconds.

2. The integrated process as in claim 1, wherein the natural gas condensate solvent has a carbon range starting at C5 or above.

3. The integrated process as in claim 1, wherein the residual oil feedstream is a straight run residue obtained from atmospheric distillation and/or vacuum distillation.

4. The integrated process as in claim 1, wherein (ii) said natural gas condensate solvent and (i) said residual oil feedstream are mixed in a (ii) to (i) ratio of from 5:1 to 25:1.

5. The integrated process as in claim 4, wherein the ratio of (ii) and (i) is from 5:1 to 15:1.

6. The integrated process of claim 1, wherein the reaction temperature is from 550°C to 630°C.

7. The integrated process of claim 1, wherein the reaction temperature is from 570°C to 610°C.

8. The integrated process of claim 1, wherein the reaction pressure is from 1 kg/cm² to 3 kg/cm².

9. The integrated process of claim 1, wherein the reaction pressure is from 1 kg/cm² to 2 kg/cm².

10. The integrated process of claim 1, wherein a riser FCC unit is used, and the contact time is from 1 second to 5 seconds.

11. The integrated process of claim 10, wherein the contact time is 1 second to 2 seconds.

12. The integrated process of claim 1, wherein a riser FCC unit is used and the catalyst:feed ratio is 2:1 to 25:1.

13. The integrated process of claim 12, wherein the catalyst:feed ratio is 2:1 to 15:1.

14. The integrated process of claim 1, wherein a downflow FCC unit is used, and the contact time is from 0.1 seconds to 5 seconds.

15. The integrated process of claim 14, wherein the contact time is from 0.2 seconds to 0.8 seconds.

16. The integrated process of claim 1, wherein a downflow FCC unit is used, and the catalyst:feed ratio is 2:1 to 30:1.

17. The integrated process of claim 16, wherein the catalyst:feed ratio is 2:1 to 20:1.

## Patentansprüche

1. Integrierter Prozess zur Erzeugung von propylenhaltigen leichten Olefinen, umfassend, dass:
(a) (i) ein Restölzustrom und/oder (ii) ein Erdgaskondensatlösemittel gemischt werden, wobei (ii) und (i) in einem Mengenverhältnis von 5:1 bis 50:1 und unter Bedingungen gemischt werden, die ausreichend sind, dass sich zumindest ein Anteil von Asphaltenen niederschlägt, um (iii) ein Gemisch aus Lösemittel- und deasphaltiertem Restöl und (iv) niedergeschlagenen Asphaltenen zu erzeugen;
(b) (iii) katalytischen Fließbettcrackreaktionen ausgesetzt wird, wobei sowohl das Lösemittel als auch das deasphaltierte Restöl von (iii) als Reaktanden in der katalytischen Fließbettcrackreaktion dienen, wobei die katalytischen Fließbettcrackreaktionen ausgeführt werden:
- in einer Steigrohr-FCC-Einheit mit einem Katalysator bei einem Verhältnis von Katalysator zu (iii) von 2:1 bis 40:1, einer Reaktionstemperatur von 500 Grad °C bis 650 Grad °C; einem Reaktionsdruck von 1 kg/cm² bis 5 kg/cm² und einer Kontaktzeit von 0,7 Sekunden bis 10 Sekunden, oder
- in einer Abstrom-FCC-Einheit mit einem Katalysator bei einem Verhältnis von Katalysator zu (iii) von 2:1 bis 40:1, einer Reaktionstemperatur von 500 Grad °C bis 650 Grad °C, einem Reaktionsdruck von 1 kg/cm² bis 5 kg/cm² und einer Kontaktzeit von 0,1 Sekunden bis 10 Sekunden.

2. Integrierter Prozess nach Anspruch 1, wobei das Erdgaskondensatlösemittel einen Kohlenstoffbereich aufweist, der bei C5 oder darüber beginnt.

3. Integrierter Prozess nach Anspruch 1, wobei der Restölzustrom ein Destillationsrest ist, der aus atmosphärischer Destillation und/oder Vakuumdestillation erhalten wird.

4. Integrierter Prozess nach Anspruch 1, wobei (ii) das Erdgaskondensatlösemittel und (i) der Restölzustrom in einem Verhältnis von (ii) zu (i) von 5:1 bis 25:1 gemischt werden.

5. Integrierter Prozess nach Anspruch 4, wobei das Verhältnis von (ii) zu (i) zwischen 5:1 bis 15:1 liegt.

6. Integrierter Prozess nach Anspruch 1, wobei die Reaktionstemperatur zwischen 550 Grad °C und 630 Grad °C liegt.

7. Integrierter Prozess nach Anspruch 1, wobei die Reaktionstemperatur zwischen 570 Grad °C und 610 Grad °C liegt.

8. Integrierter Prozess nach Anspruch 1, wobei der Reaktionsdruck zwischen 1 kg/cm² bis 3 kg/cm² liegt.

9. Integrierter Prozess nach Anspruch 1, wobei der Reaktionsdruck zwischen 1 kg/cm² bis 2 kg/cm² liegt.

10. Integrierter Prozess nach Anspruch 1, wobei eine Steigrohr-FCC-Einheit verwendet wird und die Kontaktzeit zwischen 1 Sekunde und 5 Sekunden liegt.

11. Integrierter Prozess nach Anspruch 10, wobei die Kontaktzeit 1 Sekunde bis 2 Sekunden beträgt.

12. Integrierter Prozess nach Anspruch 1, wobei eine Steigrohr-FCC-Einheit verwendet ist und das Verhältnis von Katalysator zu Zufuhr 2:1 bis 25:1 ist.

13. Integrierter Prozess nach Anspruch 12, wobei das Verhältnis von Katalysator zu Zufuhr 2:1 bis 15:1 beträgt.

14. Integrierter Prozess nach Anspruch 1, wobei eine Abstrom-FCC-Einheit verwendet ist und die Kontaktzeit zwischen 0,1 Sekunden bis 5 Sekunden liegt.

15. Integrierter Prozess nach Anspruch 14, wobei die Kontaktzeit zwischen 0,2 Sekunden bis 0,8 Sekunden liegt.

16. Integrierter Prozess nach Anspruch 1, wobei eine Abstrom-FCC-Einheit verwendet wird und das Verhältnis von Katalysator zu Zustrom 2:1 bis 30:1 beträgt.

17. Integrierter Prozess nach Anspruch 16, wobei das Verhältnis von Katalysator zu Zufuhr 2:1 bis 20:1 ist.

## Revendications

1. Procédé intégré pour la production d'oléfines légères contenant du propylène, consistant à :
(a) mélanger (i) un flux de charge de pétrole résiduel et (ii) un solvant de condensat de gaz naturel, dans lequel (ii) et (i) sont mélangés selon un rapport de 5:1 à 50:1, en quantités et dans des conditions suffisantes pour précipiter au moins une fraction d'asphaltènes pour produire (iii) un mélange de solvant et de pétrole résiduel désasphalté, et (iv) des asphaltènes précipités ;
(b) soumettre (iii) à des réactions de craquage catalytique fluidisé, dans lequel à la fois le solvant et le pétrole résiduel désasphalté de (iii) servent de réactifs dans la réaction de craquage catalytique fluidisé, dans lequel lesdites réactions de craquage catalytique fluidisé sont effectuées :
- dans une unité de craquage catalytique fluidisé, FCC, à colonne montante, avec un catalyseur, à un rapport catalyseur sur (iii) de 2:1 à 40:1, une température de réaction de 500 °C à 650 °C, une pression de réaction de 1 kg/cm² à 5 kg/cm² et un temps de contact de 0,7 seconde à 10 secondes, ou
- dans une unité de FCC à courant descendant avec un catalyseur, à un rapport catalyseur sur (iii) de 2:1 à 40:1, une température de réaction de 500 °C à 650 °C, une pression de réaction de 1 kg/cm² à 5 kg/cm² et un temps de contact de 0,1 seconde à 10 secondes.

2. Procédé intégré selon la revendication 1, dans lequel le solvant de condensat de gaz naturel possède une plage de carbone débutant à C5 ou plus.

3. Procédé intégré selon la revendication 1, dans lequel le flux de charge de pétrole résiduel est un résidu de distillation directe obtenu à partir d'une distillation atmosphérique et/ou d'une distillation sous vide.

4. Procédé intégré selon la revendication 1, dans lequel (ii) ledit solvant de condensat de gaz naturel et (i) ledit flux de charge de pétrole résiduel sont mélangés dans un rapport (ii) sur (i) de 5:1 à 25:1.

5. Procédé intégré selon la revendication 4, dans lequel le rapport de (ii) et (i) est de 5:1 à 15:1.

6. Procédé intégré selon la revendication 1, dans lequel la température de réaction est de 550 °C à 630 °C.

7. Procédé intégré selon la revendication 1, dans lequel la température de réaction est de 570 °C à 610 °C.

8. Procédé intégré selon la revendication 1, dans lequel la pression de réaction est de 1 kg/cm² à 3 kg/cm².

9. Procédé intégré selon la revendication 1, dans lequel la pression de réaction est de 1 kg/cm² à 2 kg/cm².

10. Procédé intégré selon la revendication 1, dans lequel une unité de FCC à colonne montante est utilisée et le temps de contact est de 1 seconde à 5 secondes.

11. Procédé intégré selon la revendication 10, dans lequel le temps de contact est de 1 seconde à 2 secondes.

12. Procédé intégré selon la revendication 1, dans lequel une unité de FCC à colonne montante est utilisée et le rapport catalyseur:charge est de 2:1 à 25:1.

13. Procédé intégré selon la revendication 12, dans lequel le rapport catalyseur:charge est de 2:1 à 15:1.

14. Procédé intégré selon la revendication 1, dans lequel une unité de FCC à courant descendant est utilisée et le temps de contact est de 0,1 seconde à 5 secondes.

15. Procédé intégré selon la revendication 14, dans lequel le temps de contact est de 0,2 seconde à 0,8 secondes.

16. Procédé intégré selon la revendication 1, dans lequel une unité de FCC à courant descendant est utilisée et le rapport catalyseur:charge est de 2:1 à 30:1.

17. Procédé intégré selon la revendication 16, dans lequel le rapport catalyseur:charge est de 2:1 à 20:1.
